# EUROPÄISCHE PATENTANMELDUNG

(11) **EP 4 702 994 A1**
(43) Veröffentlichungstag der Anmeldung: **04.03.2026**
(21) Anmeldenummer: 24197716.4
(22) Anmeldetag: 30.08.2024
(51) Int. Cl.: A61L 2/20, A61L 2/22

(54) **VERFAHREN UND VORRICHTUNG ZUM DEKONTAMINIEREN EINER KONTROLLIERTEN UMGEBUNG MIT EINEM DEKONTAMINATIONSMITTEL**

(71) Anmelder: TT Innovation AG, 6343 Rotkreuz (CH)
(72) Erfinder: STOKMAN, Petrus Henricus Maria, 4310 Rheinfelden (CH); THOMMEN, Alfred, 4053 Basel (CH)
(74) Vertreter: Mertzlufft-Paufler, Cornelius

(57) **Zusammenfassung**

Die Erfindung schlägt somit ein Verfahren zum Dekontaminieren (4') einer kontrollierten Umgebung (1) mit einem stabilisatorenfreien Dekontaminationsmittel (4) vor, wobei das Dekontaminationsmittel (4) in einer unmittelbaren Umgebung (9) der kontrollierten Umgebung (1) erzeugt und/oder umgehend nach einer Erzeugung, insbesondere über eine als Ultraschalldüse (15) ausgebildete Schnittstelle (3), in die kontrollierte Umgebung (1) eingebracht wird.

## Beschreibung

Die Erfindung betrifft ein Verfahren zum Dekontaminieren einer kontrollierten Umgebung mit einem Dekontaminationsmittel, sowie eine kontrollierte Umgebung mit einer Schnittstelle, über die ein Dekontaminationsmittel in die kontrollierte Umgebung einbringbar ist. Insbesondere betrifft die Erfindung ein Verfahren und eine Vorrichtung zum Dekontaminieren eines Isolators mit Wasserstoffperoxid im Bereich pharmazeutischer Anwendungen.

### Hintergrund

Die Dekontamination in Isolatoren ist ein kritischer Prozess in verschiedenen Branchen, insbesondere in der pharmazeutischen und biotechnologischen Produktion, wo sterile Bedingungen von höchster Bedeutung sind. Isolatoren sind Systeme, die eine kontrollierte Umgebung bieten und dazu verwendet werden, um Produkte, Prozesse und/oder Personal vor Kontamination zu schützen. Die Dekontamination dieser Isolatoren stellt sicher, dass Keime, Bakterien, Viren und/oder andere, insbesondere mikrobiologische, Verunreinigungen effektiv eliminiert werden, bevor sterile und/oder aseptische und/oder kontaminationskritische Prozesse beginnen. Der Einsatz ist vor Allem im aseptischen "Fill Finish"-Bereich aber auch im Bereich der Entwicklung von Arzneimitteln für neuartige Therapien ("Advanced Therapy Medicinal Products", ATMP) und Zell- und Gentherapie denkbar sowie im Einsatz mit toxischen Substanzen.

Wasserstoffperoxid (H2O2) ist ein häufig verwendetes Dekontaminationsmittel in diesen Anwendungen. Es wird eingesetzt, weil es ein starkes Oxidationsmittel ist, das Mikroorganismen effektiv abtötet, indem es deren Zellwände und DNA zerstört. Der Einsatz von H2O2 hat den Vorteil, dass es nach der Anwendung in Wasser und Sauerstoff zerfällt und somit keine schädlichen Rückstände hinterlässt oder über geeignete Katalysatoren effizient und kontrolliert abgebaut werden kann.

In der aktuellen Praxis zur Dekontamination von Isolatoren wird Wasserstoffperoxid (H2O2) in verschiedenen Konzentrationen, vorzugsweise von 35 % oder nahezu 50 % verwendet, das kommerziell erhältlich ist. Um die Stabilität während der Lagerung und des Transports zu gewährleisten, wird dieses H2O2 mit Stabilisatoren versetzt.

Die Dekontamination erfolgt typischerweise durch Verdampfen oder Einspritzen von H2O2 in den Isolator. Der entstehende H2O2-Dampf und/oder das entstehende H2O2-Aerosol gelangt in alle Ecken und Oberflächen des Isolators und sorgt für eine gleichmäßige Verteilung des Desinfektionsmittels, wodurch auch schwer zugängliche Stellen erreicht werden.

Jedoch gibt es Herausforderungen bei der Verwendung von H2O2 zur Dekontamination. Die Stabilisatoren, die dem H2O2 zugefügt werden, um seine Haltbarkeit zu verlängern, können zu Ablagerungen und/oder Verunreinigungen im Pump- und Leitungssystem, sowie in der kontrollierten Umgebung, beispielsweise im Isolator, führen. Diese Verunreinigungen können die Effizienz der Pumpe beeinträchtigen und/oder den Wartungsaufwand erhöhen. Zudem kann der Transport von H2O2 durch lange Schlauchsysteme problematisch sein, da es zur Blasenbildung und teilweise zur Zersetzung des H2O2 kommen kann, was die Wirksamkeit der Dekontamination verringert.

Insgesamt ist die Dekontamination in Isolatoren ein hochspezialisierter Prozess, der sorgfältige Planung und Wartung erfordert, um die Sterilität und Sicherheit der Produktionsumgebung zu gewährleisten.

Der vorliegenden Erfindung liegt daher die Aufgabe zugrunde, die Dekontamination kontrollierter Umgebungen nutzerfreundlicher zu gestalten.

### Zusammenfassung der Erfindung

Die Lösung dieser Aufgabe besteht bei dem Verfahren der eingangs erwähnten Art insbesondere darin, dass das Verfahren zum Dekontaminieren einer kontrollierten Umgebung, insbesondere eines Isolators, mit einem Dekontaminationsmittel, insbesondere Wasserstoffperoxid, umfasst, dass das Dekontaminationsmittel stabilisatorenfrei ist und in die kontrollierte Umgebung eingebracht wird.

Dieser Aspekt der Erfindung beschreibt damit ein Verfahren, um eine kontrollierte bzw. geschützte Umgebung, wie beispielsweise einen Isolator, eine Werkbank, eine Glove Box, eine Containment-Anlage oder einen Reinraum, zu dekontaminieren. Dabei wird ein Dekontaminationsmittel in die kontrollierte bzw. geschützte Umgebung eingebracht. Das Dekontaminationsmittel ist bevorzugt Wasserstoffperoxid. Das Dekontaminationsmittel, bzw. das Wasserstoffperoxid, zeichnet sich dadurch aus, dass es frei von Stabilisatoren ist. Der Einsatz ist vor Allem im aseptischen "Fill Finish"-Bereich aber auch im Bereich der Entwicklung von Arzneimitteln für neuartige Therapien ("Advanced Therapy Medicinal Products", ATMP) und Zell- und Gentherapie vorgesehen sowie im Einsatz mit toxischen Substanzen.

Als Stabilisator wird dabei insbesondere eine Substanz verstanden, die einer chemischen Verbindung, hier dem Dekontaminationsmittel, insbesondere H2O2, zugesetzt wird, um deren Stabilität zu erhöhen und unerwünschte chemische Reaktionen wie Zersetzung, Oxidation und/oder Polymerisation zu verhindern. Stabilisatoren fungieren dabei, indem sie die Reaktionsfähigkeit der Verbindung gegenüber äußeren Einflüssen wie Licht, Wärme und/oder Katalysatoren verringern.

Im Falle von Wasserstoffperoxid verhindern Stabilisatoren die Zersetzung durch Bindung von Katalysatoren (wie Metallionen) oder durch Schutz vor äußeren Einflüssen wie Licht.

Typische Stabilisatoren für Wasserstoffperoxid sind: (i) Phosphorsäure oder ihre Salze (z.B. Natriumphosphat): Diese binden Metallionen, die als Katalysatoren wirken könnten. (ii) Natriumstannat: Dieses stabilisiert Wasserstoffperoxid ebenfalls durch Bindung von Katalysatoren. (iii) Chelatbildner wie Ethylendiamintetraessigsäure (EDTA): Diese binden spezifisch Metallionen, die die Zersetzung fördern. (iv) Natriumkieselsäure: Diese kann zur Stabilisierung beitragen, indem sie eine Barriere gegen Zersetzung bildet.

Bei der vorliegenden Erfindung ist das Dekontaminationsmittel insbesondere frei von diesen und ähnlichen, bzw. ähnlich wirkenden Stabilisatoren.

Vorteilhafte Aspekte dieses erfindungsgemäßen Verfahrens beinhalten, dass das stabilisatorenfreie Dekontaminationsmittel keine Rückstände in einem Zuführsystem des Dekontaminationsmittels in die kontrollierte Umgebung und/oder in der kontrollierten Umgebung, insbesondere dem Isolator, hinterlässt. Dadurch wird der Aufwand nötiger Reinigungsschritte minimiert und das Dekontaminationsverfahren vereinfacht. Zudem wird die Haltbarkeit der Anlagen hierdurch erhöht. Weiterhin wird die Reproduzierbarkeit des Dekontaminationsverfahrens erhöht. Beispielsweise sind Zusammensetzung und Konzentration der Stabilisatoren dem Anwender häufig unbekannt, was einen negativen Einfluss auf die Reproduzierbarkeit des Dekontaminationsverfahrens haben kann. Auch wird die Sicherheit im Betrieb der Anlage und sowie während vor- und/oder nachgelagerter Prozesse, insbesondere im Bereich des Supply Chain Managements, verbessert.

Vorzugsweise wird das Dekontaminationsmittel bei der vorliegenden Erfindung in einer unmittelbaren Umgebung der kontrollierten Umgebung erzeugt.

Wie oben beschrieben dienen Stabilisatoren dazu, eine Zersetzung des Dekontaminationsmittels, insbesondere des H2O2, zu verhindern und dessen Stabilität während Lagerung und/oder Anwendung zu gewährleisten. In der vorliegenden Erfindung wird das Dekontaminationsmittel gerade frei von diesen Stabilisatoren in die kontrollierte, zu dekontaminierende, Umgebung eingeführt. Unter "stabilisatorenfrei" wird auch verstanden, dass das H2O2 kurz vor dem Einbringen in die kontrollierte Umgebung von den Stabilisatoren mindestens nahezu befreit werden kann.

Vorzugsweise wird das Dekontaminationsmittel in unmittelbarer Umgebung der kontrollierten Umgebung hergestellt, bzw. erzeugt. Dies hat den Vorteil, dass Lagerzeiträume und/oder Transportwege kurz gehalten werden können und damit eine Zersetzung oder Qualitätsminderung des Dekontaminationsmittels zwischen Erzeugung und Verwendung verhindert und/oder stark reduziert wird.

Weiter vorzugsweise begrenzt die unmittelbare Umgebung einen Bereich, in welchem das Dekontaminationsmittel zersetzungsfrei transportiert und/oder in die kontrollierte Umgebung eingebracht werden kann. Dies reduziert nicht nur die Kosten für den Transport und die Lagerung, sondern senkt auch das Sicherheitsrisiko, da große Mengen des Stoffes nicht über weite Strecken transportiert und/oder gelagert werden müssen. Mit anderen Worten ist die unmittelbare Umgebung der Bereich, in welchem eine Zersetzung des Dekontaminationsmittels zeitlich und/oder räumlich verhindert wird. Beispielsweise sind in dem Bereich Transportwege und/oder Lagerzeiträume des Dekontaminationsmittels derart ausgestaltet, dass eine Zersetzung des Dekontaminationsmittels verhindert wird.

Eine Zeitliche Begrenzung können insbesondere wenige Stunden, z.B. 6, 12, 16 Stunden, wenige Tage, z.B. 1, 2, 3, 4, 5, 6, 7, 8, 14 Tage oder weniger als 30 Tage sein.

Als unmitttelbare Umgebung kann insbesondere derselbe Raum, dasselbe oder benachbarte Gebäude, dieselbe Stadt, dieselbe Region, in welchem der kontrollierte Raum sich befindet, gemeint sein, oder auch ein Umkreis von weniger als 15km, 25km 50km, 100km oder 200km gemeint sein.

In einer Ausführungsform ist vorgesehen, dass das Dekontaminationsmittel elektrochemisch erzeugt wird. Ein Vorteil der elektrochemischen Erzeugung des Dekontaminationsmittels, insbesondere des Wasserstoffperoxids, ist die Umweltfreundlichkeit dieses Verfahrens. Im Gegensatz zu herkömmlichen Methoden, wie dem Anthraquinon-Verfahren, entstehen bei der elektrochemischen Produktion keine schädlichen Nebenprodukte. Die Reaktion benötigt lediglich Wasser, Sauerstoff und Elektrizität, was den Prozess wesentlich sauberer und nachhaltiger macht.

Darüber hinaus ist die elektrochemische Produktion energieeffizient. Der Prozess kann unter milden Bedingungen, wie Raumtemperatur und Umgebungsdruck, durchgeführt werden, was den Energiebedarf im Vergleich zu anderen Produktionsmethoden erheblich senkt.

Ein weiterer Vorteil liegt in der Flexibilität und Skalierbarkeit der Methode. Sie kann leicht angepasst werden, um sowohl kleine Mengen für spezielle Anwendungen als auch industrielle Mengen zu produzieren, je nach Bedarf.

Schließlich führt die geringere Entstehung von Nebenprodukten zu einem Endprodukt von höherer Reinheit, was besonders in der Pharmaindustrie von Bedeutung ist, wo hohe Qualitätsanforderungen bestehen.

Die vorliegende Erfindung beschränkt sich jedoch nicht auf elektrochemische Verfahren, es kann auch H2O2 mit jedem anderem der Fachperson bekannten Verfahren gewonnen und eingesetzt werden.

In einer weiteren Ausführungsform wird das Dekontaminationsmittel nach der Erzeugung auf eine gewünschte Konzentration eingestellt, vorzugsweise aufkonzentriert.

Wirkung und Sicherheit des Dekontaminationsmittels können von seiner Konzentration abhängen. Bei zu niedriger Konzentration kann H₂O₂ nicht die gewünschte Wirkung erzielen. Beispielsweise kann die Wirksamkeit des Dekontaminationsmittels vermindert sein, wenn dessen Konzentration zu gering ist. Auf der anderen Seite kann eine zu hohe Konzentration von Dekontaminationsmitteln sicherheitsrelevante Risiken bergen.

Es ist daher von Vorteil, das Dekontaminationsmittel nach Erzeugung auf eine gewünschte Konzentration einzustellen, um die Wirksamkeit und Sicherheit der Anwendung zu gewährleisten.

Als ein weiterer möglicherweise eigenständiger Aspekt besteht die Lösung der eingangs genannten Aufgabe bei dem Verfahren der eingangs erwähnten Art alternativ oder zusätzlich insbesondere darin, dass nur eine für den Dekontaminationsprozess der kontrollierten Umgebung benötigte Menge des Dekontaminationsmittels erzeugt wird.

Die benötigte Menge beinhaltet dabei Verluste oder Produktionsunsicherheiten oder Sicherheitsmargen für den Dekontaminationsprozess oder kann als benötigte Menge zur Durchführung von mehreren Dekontaminationsprozessen verstanden werden.

Es ist vorteilhaft, nur die benötigte Menge H₂O₂ herzustellen, um die Effizienz zu maximieren, Kosten und Risiken zu minimieren und die Umweltbelastung zu reduzieren:
Erstens kann sich das Dekontaminationsmittel, insbesondere H₂O₂, zersetzen, insbesondere unter Einfluss von Licht, Wärme, Oberflächenkontakt, Luftkontakt, Kontakt zu anderen Medien, oder Verunreinigungen. Wenn mehr H₂O₂ als nötig hergestellt wird, besteht das Risiko, dass es vor der Verwendung an Wirksamkeit verliert, was zu Verschwendung führt. Die Produktion genau der benötigten Menge minimiert dieses Risiko und stellt sicher, dass das H₂O₂ in seiner vollen Konzentration und Wirksamkeit genutzt werden kann.

Zweitens reduziert die Herstellung nur der erforderlichen Menge die Lager- und/oder Transportkosten.

Dekontaminationsmittel können spezielle Lagerungsbedingungen erfordern, da sie in höheren Konzentrationen gefährlich sein kann und Druckaufbau durch Zersetzung zu Explosionen führen kann. Wenn nur die benötigte Menge hergestellt wird, ist die Notwendigkeit, größere Mengen sicher zu lagern und/oder zu transportieren, geringer, was sowohl die Kosten als auch die Sicherheitsrisiken verringert. Insbesondere auch, da nicht oder nur kurzzeitig H2O2 von Personen gehandhabt werden muss.

Darüber hinaus minimiert die bedarfsgerechte Produktion die Umweltbelastung. Es entsteht weniger überschüssiges Material, das entsorgt oder behandelt werden muss, was die Umweltauswirkungen verringert. Außerdem wird durch die Vermeidung von Überproduktion Ressourcen wie Energie und Rohstoffe eingespart.

Als ein weiterer möglicherweise eigenständiger Aspekt besteht die Lösung der eingangs genannten Aufgabe bei dem Verfahren der eingangs erwähnten Art alternativ oder zusätzlich insbesondere darin, dass das Dekontaminationsmittel in der oder einer unmittelbaren Umgebung der kontrollierten Umgebung erzeugt wird und vorzugsweise mittels Ultraschall von einer flüssigen Form in eine gasförmige Form überführt wird und in die kontrollierte Umgebung, insbesondere über eine Ultraschalldüse, eingebracht wird.

Die Verwendung von Ultraschall, insbesondere von Ultraschalldüsen oder anderen durch Ultraschall geschützte Evaporationselemente oder -komponenten, bietet mehrere Vorteile, darunter die Fähigkeit zur extrem feinen Zerstäubung von Flüssigkeiten, was eine gleichmäßige und präzise Verteilung ermöglicht und der Kondensation vorbeugt. Sie bieten eine genaue Steuerung der Tröpfchengröße und Sprührate, was Materialeinsparungen und Effizienzsteigerungen ermöglicht. Zudem ist sie energieeffizienter, da weniger Druck erforderlich ist, und vielseitig einsetzbar, da sie eine breite Palette von Flüssigkeiten verarbeiten können, ohne zu verstopfen.

In einer Ausführungsform wird das Dekontaminationsmittel in die kontrollierte Umgebung eingespritzt und/oder während des Einbringens verdampft.

Das Einsprühen und/oder Verdampfen des Dekontaminationsmittels sorgt für eine gleichmäßige Verteilung der Flüssigkeit, was zu homogenen Beschichtungen oder Raumfeuchtigkeitsverteilung führt.

Eine feine Zerstäubung beim Einsprühen beschleunigt die Verdampfung, was für schnelle Prozesse vorteilhaft ist. Zudem ermöglicht das Einsprühen eine präzise Dosierung der Flüssigkeit und reduziert Materialabfall, was Kosten spart und umweltfreundlicher ist. In chemischen Prozessen kann es auch die Reaktionsgeschwindigkeit verbessern, indem es eine gleichmäßige Verteilung der Reaktanten fördert.

Das Verdampfen des Dekontaminationsmittels ist technologisch einfacher und erfordert keine spezielle Ausrüstung, was die Kosten senken kann. Materialverluste durch Tröpfchenbildung werden vermieden, da keine Zerstäubung stattfindet, was besonders bei teuren oder empfindlichen Flüssigkeiten vorteilhaft ist. Schließlich ermöglicht es eine präzise Steuerung der Verdampfungsgeschwindigkeit und verhindert gleichzeitig unnötige Tröpfchenbildung.

Die Einbringung des Dekontaminationsmittels erfolgt typischerweise über eine Schnittstelle. Das H2O2 kann auch durch ein anderes dem Fachmann bekanntes Verfahren der Luft zugesetzt werden. Durch den Einsatz von Ventilatoren, ein bereits mit Druck beaufschlagtes Einbringen oder durch Eigendynamik und Schwerkraft gelangt das Dekontaminationsmittel so an alle Oberflächen der kontrollierten Umgebung.

In einer alternativen Ausführungsform wird das Dekontaminationsmittel nach dem Einbringen innerhalb der kontrollierten Umgebung, insbesondere homogen, verteilt.

Ein Vorteil der, insbesondere homogenen, Verteilung des Dekontaminationsmittels liegt darin, dass es vorzugsweise gleichmäßig auf der gesamten Oberfläche innerhalb der kontrollierten Umgebung wirkt und/oder zu diesen befördert werden kann. Dies gewährleistet eine vollständige Abdeckung und effektive Dekontamination oder Desinfektion, da alle Bereiche der Oberfläche gleichmäßig behandelt werden. Eine gleichmäßige Verteilung verhindert Materialverschwendung und Rückstände und trägt zur Sicherheit bei, indem sie sicherstellt, dass alle Kontaminanten entfernt werden. Die homogene Verteilung kann beispielsweise mittels eines Ventilators und/oder mittels Druckluft erfolgen.

In einer alternativen Ausführungsform ist vorgesehen, dass das Dekontaminationsmittel (4) in der kontrollierten Umgebung stabilisatorenfrei wird, insbesondere von Stabilisatoren befreit wird, oder dass das Dekontaminationsmittel vor Einbringen in die kontrollierte Umgebung stabilisatorenfrei wird, insbesondere von Stabilisatoren befreit wird.

Mit anderen Worten kann das Dekontaminationsmittel zunächst Stabilisatoren enthalten, welche aber vor dem Verteilen des Dekontaminationsmittels in der kontrollierten Umgebung entfernt wird. Das Entfernen kann vor Einbringen des Dekontaminationsmittels in beispielsweise den Isolator erfolgen. Alternativ kann das Entfernen der Stabilisatoren in einem Aufbereitungsschritt des Dekontaminationsmittels in der kontrollierten Umgebung erfolgen. Das Entfernen der Stabilisatoren aus dem Dekontaminationsmittel, bzw. das Trennen des Dekontaminationsmittels von den Stabilisatoren, kann durch chemische und/oder physikalische Prozesse erfolgen. Beispielsweise kann dieser Aufbereitungsschritt zusätzlich das Auf- oder Herunterkonzentrieren des Dekontaminationsmittels umfassen.

Dadurch kann ein zunächst stabilisiertes Dekontaminationsmittel verwendet werden, wobei oben beschriebene Vorteile der Nutzung eines stabilisatorenfreien Dekontaminationsmittels zur Dekontamination der kontrollierten Umgebung erhalten bleiben, da weiterhin nur stabilisatorenfreies Dekontaminationsmittel zur Dekontamination verwendet wird.

In einer weiteren Ausführungsform ist vorgesehen, dass das Dekontaminationsmittel vor dem Einbringen in die kontrollierte Umgebung stabilisatorenfrei zwischengespeichert wird. Dadurch kann das Dekontaminationsmittel beispielsweise nach Erzeugung zunächst gesammelt und/oder aufbereitet, beispielsweise aufkonzentriert, werden. Dadurch kann beispielsweise ein niedriger Durchsatz der das Dekontaminationsmittel erzeugenden Einheit/en kompensiert werden. Der Zwischenspeicher kann dabei beispielsweise von der das Dekontaminationsmittel bereitstellenden Einheit gebildet sein.

Als ein weiterer möglicherweise eigenständiger Aspekt besteht die Lösung der eingangs genannten Aufgabe in einer kontrollierte Umgebung, insbesondere einem Isolator, mit wenigstens einer Schnittstelle, über die ein Dekontaminationsmittel, insbesondere Wasserstoffperoxid, in die kontrollierte Umgebung einbringbar ist und insbesondere zum Durchführen eines wie oben beschriebenen Verfahrens geeignet ist, wobei die Schnittstelle mit einer das Dekontaminationsmittel erzeugenden Einheit und/oder einer das Dekontaminationsmittel stabilisatorenfrei bereitstellenden Einheit verbindbar ist.

Dieser Aspekt der Erfindung beschreibt einen kontrollierte bzw. geschützte Umgebung, wie beispielsweise einen Isolator, eine Werkbank, eine Glovebox, eine Containment-Anlage und/oder einen Reinraum. Diese kontrollierte Umgebung umfasst mindestens eine Schnittstelle, über die ein Dekontaminationsmittel in den kontrollierten Raum innerhalb der kontrollierten Umgebung einbringbar ist. Die Anzahl der Schnittstellen wird je nach Ausgestaltung der kontrollierten Umgebung, beispielsweise nach Ausführung des Isolators, definiert. Die mindestens eine Schnittstelle kann Düsen oder Sprühköpfe beinhalten, die das Dekontaminationsmittel möglichst gleichmäßig verteilen. Weiter ist die Schnittstelle derart ausgestaltet, dass eine Einheit verbindbar ist, welche das Dekontaminationsmittel bereitstellt und/oder erzeugt. Die Einheit kann somit beispielsweise Lagerungsbehältnisse des Dekontaminationsmittel umfassen und/oder eine Erzeugungseinheit insbesondere elektrochemische Reaktionszellen umfassen, welche das Dekontaminationsmittel erzeugen. Damit kann das Dekontaminationsmittel unmittelbar nach Erzeugung in die kontrollierte Umgebung eingebracht werden, ohne dass ein separater Transport nötig ist. Weiter kann das Dekontaminationsmittel nach Erzeugung ohne Kontakt zur (nicht-kontrollierten) Umgebung eingebracht werden, was das Kontaminationsrisiko des Dekontaminationsmittels durch Umfüllen, Transport und/oder Lagerung stark vermindert. Dadurch wird auch die Reproduzierbarkeit des Dekontaminationsprozesses innerhalb der kontrollierten Umgebung erhöht.

In einer Ausführungsform ist vorgesehen, dass die Schnittstelle dazu ausgebildet ist, das Dekontaminationsmittel vorzugsweise mittels Ultraschall von einer flüssigen Form in eine gasförmige Form zu überführen und in die kontrollierte Umgebung, insbesondere über eine Ultraschalldüse, einzubringen.

Wie oben beschrieben ermöglicht Verwendung von Ultraschall, insbesondere von Ultraschalldüsen, eine extrem feinen Zerstäubung des Dekontaminationsmittels, was eine gleichmäßige und präzise Verteilung ermöglicht. Sie bieten eine genaue Steuerung der Tröpfchengröße und Sprührate, was Materialeinsparungen und Effizienzsteigerungen ermöglicht. Zudem ist sie energieeffizienter, da weniger Druck erforderlich ist, und vielseitig einsetzbar, da sie eine breite Palette von Dekontaminationsmitteln verarbeiten können, ohne zu verstopfen.

In einer Ausführungsform umfasst die kontrollierte Umgebung zusätzlich einen Zwischenspeicher, in dem das Dekontaminationsmittel, insbesondere nach dessen Erzeugung, stabilisatorenfrei zwischengespeichert werden kann. Dadurch kann das Dekontaminationsmittel beispielsweise nach Erzeugung zunächst gesammelt und/oder aufbereitet, beispielsweise aufkonzentriert, werden. Der Zwischenspeicher kann dabei beispielsweise von der das Dekontaminationsmittel bereitstellenden Einheit gebildet sein. Insbesondere kann die Aufkonzentration, oder Herabkonzentration, des Dekontaminationsmittels in dem Zwischenspeicher erfolgen. Der Zwischenspeicher kann Teil der Bereitstellungseinheit sein, oder dezentral in der unmittelbaren Umgebung der kontrollierten Umgebung verortet sein. Dabei kann die kontrollierte Umgebung, insbesondere der Isolator, zentral von einem Zwischenspeicher versorget werden, oder jeder Schnittstelle je ein Zwischenspeicher zugeordnet werden.

In einer weiteren Ausführungsform ist die Schnittstelle über eine Leitung mit der das Dekontaminationsmittel erzeugenden Einheit und/oder mit der das Dekontaminationsmittel stabilisatorenfrei bereitstellenden Einheit verbunden. Damit kann das Dekontaminationsmittel unmittelbar ohne Kontakt zur Atmosphäre und Umgebung frei von Verunreinigungen in die kontrollierte Umgebung transportiert werden. Für den Transport von Dekontaminationsmitteln wie Wasserstoffperoxid werden chemisch resistente Leitungen und Schlauchverbindungen verwendet. Beispielsweise können die Leitungen PTFE-Schläuche, Silikonschläuche, FEP- und PVDF-Schläuche, oder Edelstahlrohre, insbesondere hochlegierte Edelstahlrohre, umfassen insbesondere hochlegierter Edelstahl, gut geeignet.

Die Leitung kann ebenfalls chemisch resistente Verbindungselemente, wie Fittings und Dichtungen aus PTFE, umfassen, um sichere Verbindungen zu gewährleisten.

Es kann dabei beispielsweise auch vorgesehen sein, dass die Leitung dabei geometrisch höher als die Schnittstelle ausgebildet ist. Somit kann ein Abpumpen bzw. ein Absaugen des Dekontaminationsmittels nach dem Dekontaminieren der kontrollierten Umgebung erleichtert werden.

Ein weiterer möglicherweise eigenständiger Aspekt der Erfindung liegt in einer Verwendung einer wie oben beschriebenen kontrollierten Umgebung zum Durchführen eines wie oben beschriebenen Verfahrens.

Die Vorteile liegen wie oben geschrieben vor.

Die Erfindung wird nun anhand von Ausführungsbeispielen näher beschrieben, ist jedoch nicht auf die Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen des Ausführungsbeispiels.

Es zeigt, in jeweils stark vereinfachter Darstellung,

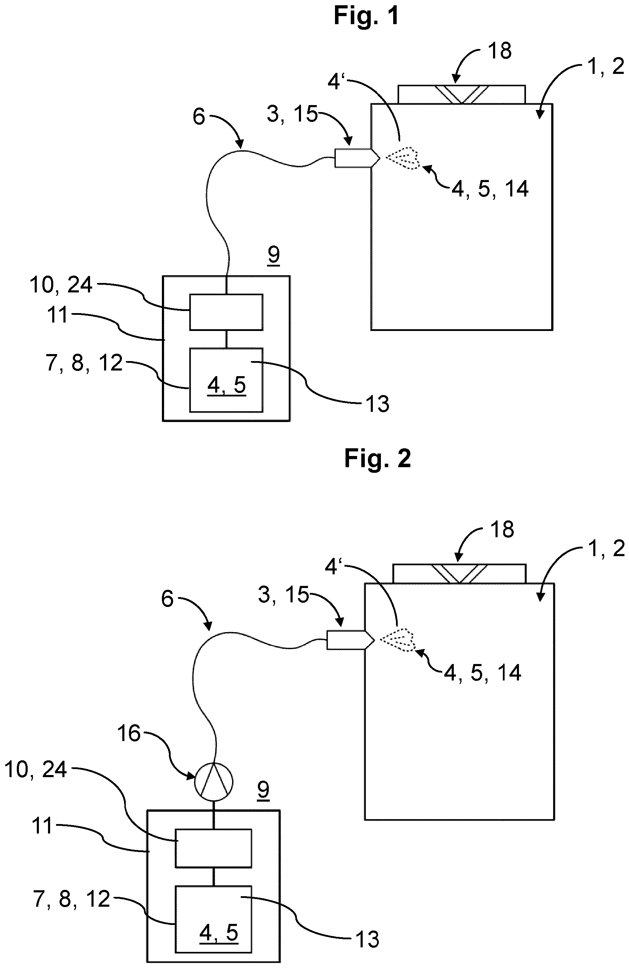

- Fig. 1: eine schematische, zweidimensionale Darstellung einer kontrollierten Umgebung mit Filtereinheit und einer Schnittstelle, die mit einer ein Dekontaminationsmittel erzeugenden Einheit und einer das Dekontaminationsmittel aufkonzentrierenden Einheit über eine Leitung verbunden ist, wobei das Dekontaminationsmittel über die Schnittstelle in die kontrollierte Umgebung eingebracht wird,
- Fig. 2: eine schematische, zweidimensionale Darstellung einer kontrollierten Umgebung gemäß Fig. 1, wobei das von der Einheit erzeugte Dekontaminationsmittel mittels einer Pumpe über die Leitung zur Schnittstelle befördert und über die Schnittstelle in die kontrollierte Umgebung eingebracht wird.

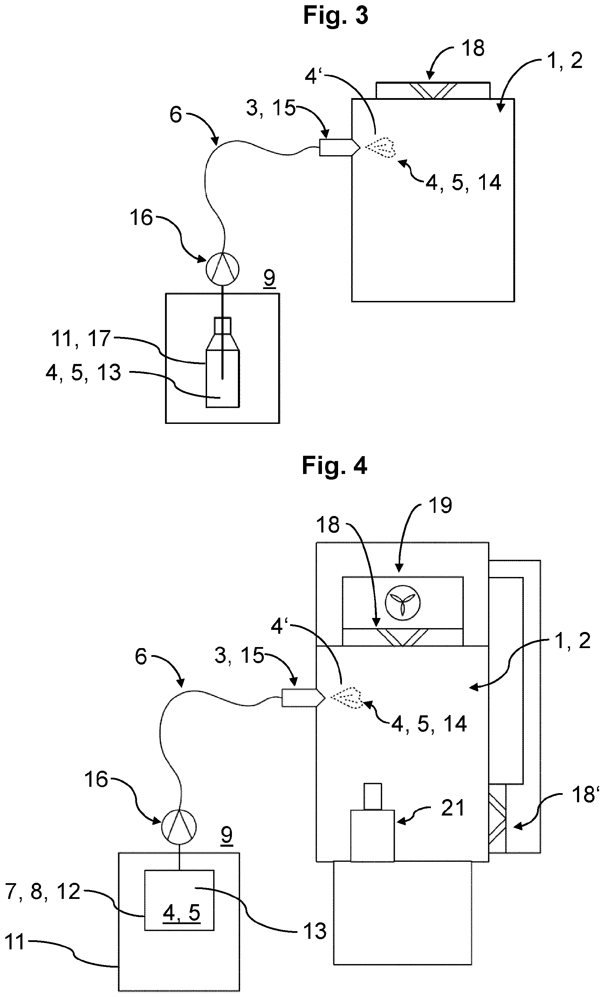

- Fig. 3: eine schematische, zweidimensionale Darstellung einer kontrollierten Umgebung gemäß Fig. 2, in die Dekontaminationsmittel eingebracht wird, wobei das Dekontaminationsmittel aus einer das Dekontaminationsmittel stabilisatorenfrei bereitstellenden Einheit bezogen wird,
- Fig. 4: eine schematische, zweidimensionale Darstellung einer kontrollierten Umgebung mit darin ausgebildetem, nach der Dekontamination zu prozessierendem Objekt und Filtereinheiten, wobei das Dekontaminationsmittel in unmittelbarer Umgebung der kontrollierten Umgebung von einer Einheit erzeugt in die kontrollierte Umgebung über eine Schnittstelle eingebracht wird,

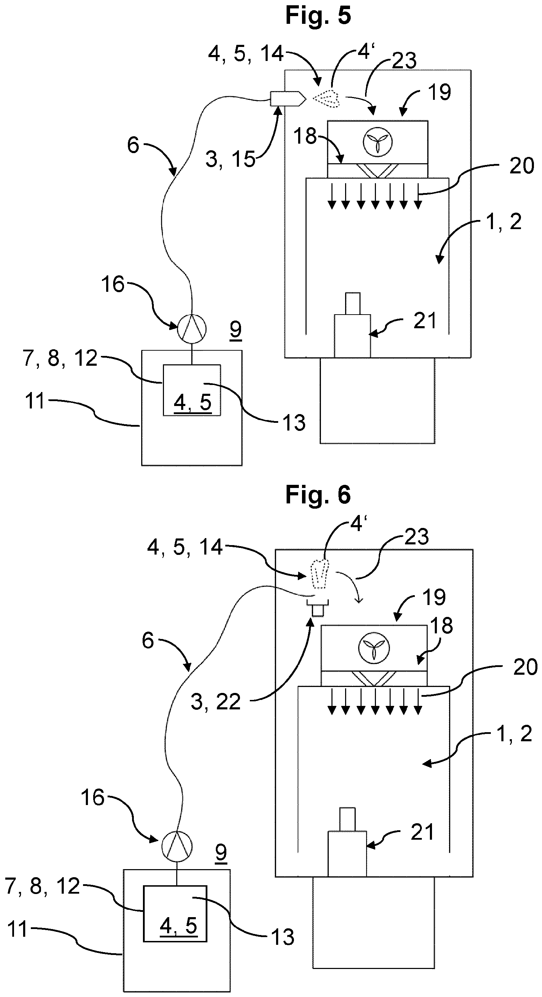

- Fig. 5: eine schematische, zweidimensionale Darstellung einer kontrollierten Umgebung, in die in unmittelbarer Umgebung der kontrollierten Umgebung erzeugtes Dekontaminationsmittel gemäß Fig. 4 eingebracht wird, wobei das Dekontaminationsmittel von einer Ventilatoreinheit innerhalb der kontrollierten Umgebung angesaugt und verteilt wird,
- Fig. 6: eine schematische, zweidimensionale Darstellung einer kontrollierten Umgebung, in die in unmittelbarer Umgebung der kontrollierten Umgebung erzeugtes Dekontaminationsmittel verdampfend eingebracht und gemäß Fig. 5 von der Ventilatoreinheit angesaugt und innerhalb der kontrollierten Umgebung verteilt wird.

Fig. 1 zeigt eine im Ganzen mit 1 bezeichnete kontrollierte Umgebung, hier einen Isolator 2. Die kontrollierte Umgebung 1 hat wenigstens eine Schnittstelle 3, über die ein Dekontaminationsmittel 4, in diesem Ausführungsbeispiel Wasserstoffperoxid (H2O2) 5, zur Dekontaminierung 4' in die kontrollierte Umgebung 1 einbringbar ist. Die Dekontaminierung 4' der kontrollierten Umgebung 1 bewirkt eine Reduzierung einer Keimbelastung, sodass ein nachfolgender Prozess, der innerhalb der kontrollierten Umgebung 1, unter geeigneten Bedingungen ausgeführt werden kann oder ein kontrollierter Raum geöffnet werden kann. So werden insbesondere mikrobiologische Verunreinigungen und/oder toxische und/oder aktive Substanzen inaktiviert.

Die kontrollierte Umgebung 1 umfasst, insbesondere für den nach der Dekontaminierung ablaufenden Prozess, eine Filtereinheit 18, die jedoch auch während der Dekontamination 4' der kontrollierten Umgebung 1, insbesondere zur Verteilung des Dekontaminationsmittels 4 innerhalb der kontrollierten Umgebung 1, eine Rolle spielen kann (siehe hierzu insbesondere die Figurenbeschreibung zu Fig. 5 und 6).

Die Schnittstelle 3 ist in dem Ausführungsbeispiel nach Fig. 1 über eine Leitung 6 mit einer das Dekontaminationsmittel 4 erzeugenden Einheit 7 verbunden. Die das Dekontaminationsmittel 4 erzeugende Einheit 7 umfasst hierbei eine elektrochemischen Zelle 8, über die das Dekontaminationsmittel 4 in einer unmittelbaren Umgebung 9 der kontrollierten Umgebung 1, hier elektrochemisch, erzeugt wird, wodurch wiederum auf eine Beimischung von Stabilisatoren in das Dekontaminationsmittel 4 verzichtet werden kann. Ein überaus vorteilhafter Aspekt ergibt sich durch stabilisatorenfreie Dekontaminationsmittel 4 ferner dadurch, dass, selbst wenn sich dieses zersetzt, keine Rückstände gebildet werden, die beispielsweise eine Verunreinigung der kontrollierten Umgebung bewirken können.

Es ist ferner eine Komponente 10 ausgebildet, hier der das Dekontaminationsmittel 4 erzeugenden Einheit 7 nachgeschaltet, über die das Dekontaminationsmittel 4 auf eine gewünschte Konzentration eingestellt, hier aufkonzentriert, werden kann. Auch eine Verdünnung des Dekontaminationsmittels ist denkbar.

In einer hier nicht dargestellten Ausführungsform könnte der erzeugenden Einheit 7 noch ein Zwischenspeicher nachgeschaltet sein, in welchem das Dekontaminationsmittel 4 vor dem Einbringen in die kontrollierte Umgebung 1 gespeichert wird. Insbesondere wenn die erzeugende Einheit 7 nicht unmittelbar die benötigte Menge Dekontaminationsmittel 4 bereitstellen kann.

Das wie in Figur 1 über die elektrochemische Zelle 8 erzeugte Dekontaminationsmittel 4 wird in diesem Ausführungsbeispiel aufkonzentriert, anschließend über die Leitung 6 zur Schnittstelle 3 transportiert und über diese in die kontrollierte Umgebung 1 eingebracht, hier eingespritzt, und, zumindest in diesem Ausführungsbeispiel, mittels eines Einspritzdrucks, beispielsweise mittels Druckluft, innerhalb der kontrollierten Umgebung 1 verteilt wird. Wie in einem weiteren Ausführungsbeispiel (bspw. Fig. 4, 5 und 6) gezeigt, kann auf eine solche Komponente 10 auch verzichtet werden.

Es kann somit gesagt werden, dass ein Verfahren zum Dekontaminieren 4` der kontrollierten Umgebung 1, hier des Isolators 2, mit dem Dekontaminationsmittel 4, hier mittels Wasserstoffperoxid (H2O2) 5, durchgeführt werden kann, wobei das Dekontaminationsmittel 4, zumindest zum Zeitpunkt des Eintritts in die kontrollierte Umgebung, stabilisatorenfrei ist und in die kontrollierte Umgebung 1 eingebracht wird.

Es sei hierbei angemerkt, dass die das Dekontaminationsmittel 4 erzeugende Einheit 7 nicht auf eine elektrochemische Zelle 8 eingeschränkt ist und alternativ oder zusätzlich weitere, aus dem Stand der Technik bekannte Dekontaminationsmittel 4 erzeugende Einheiten 7 denkbar sind.

Im gezeigten Ausführungsbeispiel bildet die das Dekontaminationsmittel 4 erzeugende Einheit 7 (mit oder ohne der die Konzentration des Dekontaminationsmittels 4 einstellbaren Komponente 10) auch eine das Dekontaminationsmittel 4 stabilisatorenfrei bereitstellende Einheit 11, da das durch die in unmittelbarer Umgebung 9 der kontrollierten Umgebung 1 erzeugte Dekontaminationsmittel 4 nicht nur erzeugt, sondern ebenso bereitgestellt und umgehend in die kontrollierte Umgebung 1 eingebracht wird, wodurch keine Stabilisatoren innerhalb des Dekontaminationsmittels 4 notwendig sind. Die unmittelbare Umgebung 9 begrenzt dabei einen Bereich, in welchem das Dekontaminationsmittel 4 zersetzungsfrei transportiert und/oder in die kontrollierte Umgebung 1 eingebracht werden kann.

Dadurch, dass die das Dekontaminationsmittel 4 erzeugende Einheit 7 mit der Komponente 10, über die wiederum die Konzentration des Dekontaminationsmittels 4 eingestellt werden kann, verbunden ist, kann somit gesagt werden, dass das Dekontaminationsmittel 4 nach der Erzeugung 12 auf eine gewünschte Konzentration eingestellt, hier aufkonzentriert, wird.

Mittels einer Verwendung der beschriebenen kontrollierten Umgebung 1 kann ferner beispielsweise ein Verfahren zum Dekontaminieren 4` der kontrollierten Umgebung 1 realisiert werden, wobei das Dekontaminationsmittel 4 in der unmittelbaren Umgebung 9 der kontrollierten Umgebung 1, beispielsweise in bereits beschriebener Art und Weise, erzeugt und von einer flüssigen Form 13 in eine gasförmige Form 14, beispielsweise mittels Ultraschall, überführt wird und in die kontrollierte Umgebung 1 eingebracht wird. Dies ist insbesondere dann vorteilhaft, wenn die Schnittstelle 3, über die das Dekontaminationsmittel 4 eingebracht wird, dabei als Ultraschalldüse 15 ausgebildet ist, da durch Ultraschalldüsen 15 eine extrem feine Zerstäubung des Dekontaminationsmittels 4 ermöglicht ist.

Ferner kann, da die Erzeugung des Dekontaminationsmittels 4 in unmittelbarer Umgebung 9 der kontrollierten Umgebung erfolgt, das Dekontaminationsmittel 4 stabilisatorenfrei in die kontrollierte Umgebung 1 eingebracht werden, wobei wiederum eine Verstopfung der Ultraschalldüse 15 durch Stabilisatoren des Dekontaminationsmittels 4 verhindert werden kann.

Es kann ferner vorgesehen sein, dass, beispielsweise über die das Dekontaminationsmittel 4 erzeugende Einheit 7, nur die Menge an Dekontaminationsmittel 4 erzeugt wird, die für den Dekontaminationsprozess, insbesondere die Dekontaminierung, der kontrollierten Umgebung 1 tatsächlich benötigt wird.

Fig. 2 zeigt im Unterschied zum vorangehenden Ausführungsbeispiel eine weitere Variante zum Dekontaminieren 4` der kontrollierten Umgebung 1. Funktionell und/oder konstruktiv zu dem vorgehenden Ausführungsbeispiel gleichartige oder identische Bauteile und Funktionseinheiten sind mit denselben Bezugszeichen bezeichnet und nicht noch einmal gesondert beschrieben.

Um das in unmittelbarer Umgebung 9 der kontrollierten Umgebung 1 erzeugte Dekontaminationsmittel 4 in die kontrollierte Umgebung 1 einzubringen, wird dieses über eine Pumpe 16 von der das Dekontaminationsmittel 4 erzeugenden Einheit 7 und der dieser nachgeschalteten Komponente 10 zum Einstellen der Konzentration durch die Leitung 6 zur Schnittstelle 3 transportiert. Über die Schnittstelle 3 wird das Dekontaminationsmittel 4 sodann in die kontrollierte Umgebung eingebracht, beispielsweise eingespritzt oder eingedüst. Es ist auch möglich, dass das Dekontaminationsmittel 4 verdampft und danach dieser Dampf in die kontrollierte Umgebung 1 geleitet wird.

Fig. 3 zeigt im Unterschied zum Ausführungsbeispiel nach Fig. 2 keine das Dekontaminationsmittel 4 erzeugende Einheit 7, sondern lediglich die oder eine das Dekontaminationsmittel 4 stabilisatorenfrei bereitstellende Einheit 11. Im gezeigten Ausführungsbeispiel ist die Schnittstelle 3 somit nicht mit der das Dekontaminationsmittel 4 erzeugenden Einheit 7, sondern mit der das Dekontaminationsmittel 4 stabilisatorenfei bereitstellenden Einheit 11, hier in Form einer Flasche 17, in welches das zuvor in unmittelbarer Umgebung 9 der kontrollierten Umgebung 1 erzeugte Dekontaminationsmittel 4 abgefüllt wurde und aus diesem beziehbar ist, verbunden.

Die Konzentration des in der Flasche 17 stabilisatorenfrei abgefüllten Dekontaminationsmittels 4 kann dabei bereits entsprechend eingestellt sein. Möglich kann auch sein, dass der das Dekontaminationsmittel 4 stabilisatorenfrei bereitstellenden Einheit 11, wie in den vorangehenden Ausführungsbeispielen gezeigt, eine entsprechende Komponente 10 zum Einstellen der Konzentration des Dekontaminationsmittels nachgeschaltet wird.

Das stabilisatorenfreie Dekontaminationsmittel 4 kann auch in diesem Ausführungsbeispiel in bereits beschriebener Art und Weise in die kontrollierte Umgebung 1, hier den Isolator 2, eingebracht werden.

Fig. 4 stellt, zumindest schematisch, einen erweiterten Aufbau der kontrollierten Umgebung 1 mit einer Ventilatoreinheit 19, die der bereits dargestellten Filtereinheit 18 vorgeschaltet ist, dar. Über die Ventilatoreinheit 19 kann, wie in der Beschreibung zu den Fig. 5 und 6 näher konkretisiert, ein Fluidstrom 20 generiert werden (in Fig. 4 nicht dargestellt), der wiederum über eine weitere Filtereinheit 18' gefiltert und zurück zur Ventilatoreinheit 19 geführt werden kann. Darüber hinaus sind ein oder mehrere Einbauten 21 innerhalb der kontrollierten Umgebung 1 ausgebildet, welche während des Dekontaminationsprozesses und/oder in einem der Dekontaminierung 4' nachfolgenden Prozess eingesetzt werden können, beispielsweise um ein pharmazeutisches und/oder biotechnologisches Erzeugnis zu befüllen, zu verarbeiten, zu bewegen, zu analysieren oder zu prozessieren. Beispielsweise kann der Einbau 21 eine Maschine sein, welche ein Behältnis prozessiert und/oder verarbeitet, beispielsweise mit einem pharmazeutischen Erzeugnis befüllt. Alternativ kann Einbau 21 auch beispielsweise eine Füllmaschine, Prozessstation oder Messeinrichtung sein. Während der Dekontaminierung 4` der kontrollierten Umgebung 1, die in bereits beschriebener Art und Weise erfolgt, ist die Ventilatoreinheit 19 inaktiv. In einer alternativen Ausführungsform kann die Ventilatoreinheit 19 auch aktiv sein.

Ein Unterscheidungsmerkmal ergibt sich im Ausführungsbeispiel nach Fig. 4 ferner dadurch, dass der das Dekontaminationsmittel 4 erzeugenden Einheit 7 keine die Konzentration des Dekontaminationsmittels 4 einstellende Komponente 10 nachgeschaltet ist. In einem nicht dargestellten Ausführungsbeispiel kann jedoch eine einstellende Komponente 10 zur Einstellung der Konzentration eingesetzt werden.

Fig. 5 zeigt ein weiteres erfindungsgemäßes Ausführungsbeispiel einer kontrollierten Umgebung 1, auch hier eines Isolators 2, über die ein bereits beschriebenes Verfahren, insbesondere das Verfahren zur Dekontaminierung 4' der kontrollierten Umgebung 1 mit einem stabilisatorenfreien Dekontaminationsmittel 4, welches in die kontrollierte Umgebung 1 eingebracht wird, durchgeführt werden kann.

Im Unterschied zum Ausführungsbeispiel gemäß Fig. 4, wird die Ventilatoreinheit 19 während der Dekontaminierung 4' der kontrollierten Umgebung 1 aktiviert, wodurch wiederum der in der Beschreibung zu Fig. 4 eingeführte Fluidstrom 20 erzeugt wird. Das Dekontaminationsmittel 4 wird hierbei zunächst von der Einheit 7 erzeugt und von der Pumpe 16 über die Leitung 6 zur Schnittstelle 3 transportiert, über die das Dekontaminationsmittel 4 in bereits beschriebener Weise in die kontrollierte Umgebung 1 eingebracht wird.

Die Schnittstelle 3 ist weiterhin derart an der kontrollierten Umgebung 1 ausgebildet, dass das eingebrachte, insbesondere eingespritzte, Dekontaminationsmittel 4 sodann von der aktivierten Ventilatoreinheit 19 angesaugt 23 und durch den Fluidstrom 20, nach einem Passieren der der Ventilatoreinheit 19 nachgeschalteten Filtereinheit 18, innerhalb der kontrollierten Umgebung 1 verteilt werden kann. Ferner wird der Fluidstrom 20 im gezeigten Ausführungsbeispiel ohne erneutes Filtrieren seitlich in der Zwischenwand / Zwischenscheibe zurückgeführt.

Fig. 6 unterscheidet sich von dem Ausführungsbeispiel nach Fig. 5 insbesondere dadurch, dass das Dekontaminationsmittel 4 über die Schnittstelle 3 nicht eingespritzt, sondern verdampfend in die kontrollierte Umgebung 1 über eine Verdampfungseinheit 22, die Bestandteil der Schnittstelle 3 sein kann, eingebracht wird. Es kann somit gesagt werden, dass das Dekontaminationsmittel 4 während des Einbringens verdampft wird. Das verdampfte Dekontaminationsmittel 4 wird anschließend von der in diesem Ausführungsbeispiel ebenfalls aktivierten Ventilatoreinheit 19 angesaugt 23, durch die Filtereinheit 18 geleitet und mit dem Fluidstrom 20 innerhalb der kontrollierten Umgebung 1 verteilt.

Die Erfindung schlägt somit ein Verfahren zum Dekontaminieren 4' einer kontrollierten Umgebung 1 mit einem stabilisatorenfreien Dekontaminationsmittel 4 vor, wobei das Dekontaminationsmittel 4 in einer unmittelbaren Umgebung 9 der kontrollierten Umgebung 1 erzeugt und umgehend in die kontrollierte Umgebung 1, insbesondere über eine als Ultraschalldüse 15 ausgebildete Schnittstelle 3, eingebracht wird.

### Bezugszeichenliste

- 1: kontrollierte Umgebung
- 2: Isolator
- 3: Schnittstelle
- 4: Dekontaminationsmittel

- 4': Dekontaminierung
- 5: Wasserstoffperoxid (H2O2)
- 6: Leitung
- 7: erzeugende Einheit
- 8: elektrochemische Zelle
- 9: unmittelbare Umgebung
- 10: Komponente
- 11: bereitstellende Einheit
- 12: Erzeugung
- 13: flüssige Form
- 14: gasförmige Form
- 15: Ultraschalldüse
- 16: Pumpe
- 17: Flasche
- 18: Filtereinheit

- 18`: Filtereinheit
- 19: Ventilatoreinheit
- 20: Fluidstrom
- 21: Einbau
- 22: Verdampfungseinheit
- 23: Ansaugung
- 24: Zwischenspeicher

## Patentansprüche

1. Verfahren zum Dekontaminieren (4') einer kontrollierten Umgebung (1), insbesondere eines Isolators (2), mit einem Dekontaminationsmittel (4), insbesondere Wasserstoffperoxid (5), **dadurch gekennzeichnet, dass** das Dekontaminationsmittel (4) stabilisatorenfrei ist und in die kontrollierte Umgebung (1) eingebracht wird.

2. Verfahren nach Anspruch 1, **dadurch gekennzeichnet, dass** das Dekontaminationsmittel (4) in einer unmittelbaren Umgebung (9) der kontrollierten Umgebung (1) erzeugt wird.

3. Verfahren nach Anspruch 2, **dadurch gekennzeichnet, dass** die unmittelbare Umgebung (9) einen Bereich begrenzt, in welchem das Dekontaminationsmittel (4) zersetzungsfrei transportiert und/oder in die kontrollierte Umgebung (1) eingebracht wird.

4. Verfahren nach einem der vorangehenden Ansprüche, **dadurch gekennzeichnet, dass** das Dekontaminationsmittel (4) elektrochemisch erzeugt wird.

5. Verfahren nach einem der Ansprüche 2 bis 4, **dadurch gekennzeichnet, dass** das Dekontaminationsmittel (4) nach der Erzeugung auf eine gewünschte Konzentration eingestellt, vorzugsweise aufkonzentriert, wird.

6. Verfahren nach dem Oberbegriff von Anspruch 1 oder nach einem der vorangehenden Ansprüche, **dadurch gekennzeichnet, dass** nur eine für den Dekontaminationsprozess (4') der kontrollierten Umgebung (1) benötigte Menge des Dekontaminationsmittels (4) erzeugt wird.

7. Verfahren nach dem Oberbegriff von Anspruch 1 oder nach einem der vorangehenden Ansprüche, **dadurch gekennzeichnet, dass** das Dekontaminationsmittel (4) in der oder einer unmittelbaren Umgebung (9) der kontrollierten Umgebung (1) erzeugt wird und vorzugsweise mittels Ultraschall von einer flüssigen Form (13) in eine gasförmige Form (14) überführt wird und in die kontrollierte Umgebung (1), insbesondere über eine Ultraschalldüse (15), eingebracht wird.

8. Verfahren nach einem der vorangehenden Ansprüche, **dadurch gekennzeichnet, dass** das Dekontaminationsmittel (4) in die kontrollierte Umgebung (1) eingespritzt und/oder während des Einbringens verdampft wird.

9. Verfahren nach einem der vorangehenden Ansprüche, **dadurch gekennzeichnet, dass** das Dekontaminationsmittel (4) nach dem Einbringen innerhalb der kontrollierten Umgebung (1), insbesondere homogen, verteilt wird.

10. Verfahren nach einem der vorangehenden Ansprüche, **dadurch gekennzeichnet, dass** das Dekontaminationsmittel (4) in der kontrollierten Umgebung (1) stabilisatorenfrei wird, insbesondere von Stabilisatoren befreit wird, oder dass das Dekontaminationsmittel (4) vor dem Einbringen in die kontrollierte Umgebung (1) stabilisatorenfrei wird, insbesondere von Stabilisatoren befreit wird.

11. Verfahren nach einem der vorangehenden Ansprüche, **dadurch gekennzeichnet, dass** das Dekontaminationsmittel (4) vor dem Einbringen in die kontrollierte Umgebung (1) stabilisatorenfrei zwischengespeichert wird.

12. Kontrollierte Umgebung (1), insbesondere ein Isolator (2), mit wenigstens einer Schnittstelle (3), über die ein Dekontaminationsmittel (4), insbesondere Wasserstoffperoxid (5), in die kontrollierte Umgebung (1) einbringbar ist, insbesondere zum Durchführen eines Verfahrens nach einem der Ansprüche 1 bis 11, **dadurch gekennzeichnet, dass** die Schnittstelle (3) mit einer das Dekontaminationsmittel (4) erzeugenden Einheit (7) und/oder einer das Dekontaminationsmittel (4) stabilisatorenfrei bereitstellenden Einheit (11) verbindbar ist.

13. Kontrollierte Umgebung (1) nach Anspruch 12, zusätzlich umfassend einen Zwischenspeicher (24), in dem das Dekontaminationsmittel (4), insbesondere nach dessen Erzeugung, stabilisatorenfrei zwischengespeichert werden kann.

14. Kontrollierte Umgebung (1) nach einem der vorangehenden Ansprüche 12 - 13, **dadurch gekennzeichnet, dass** die mindestens eine Schnittstelle (3) dazu ausgebildet ist, das Dekontaminationsmittel (4) vorzugsweise mittels Ultraschall von einer flüssigen Form (13) in eine gasförmige Form (14) zu überführen und in die kontrollierte Umgebung (1), insbesondere über eine Ultraschalldüse (15), einzubringen.

15. Kontrollierte Umgebung (1) nach einem der vorangehenden Ansprüche 12 - 14, **dadurch gekennzeichnet, dass** die mindestens eine Schnittstelle (3) über mindestens eine Leitung (6) mit der das Dekontaminationsmittel (4) erzeugenden Einheit (7) und/oder mit der das Dekontaminationsmittel (4) stabilisatorenfrei bereitstellenden Einheit (11) verbunden ist.

16. Verwendung einer kontrollierten Umgebung (1) nach einem der Ansprüche 10 - 15 zum Durchführen eines Verfahrens nach einem der Ansprüche 1 bis 11.
